(19) Europäisches Patentamt European Patent Office Office européen des brevets

(11) **EP 3 014 268 B1**

(12) **EUROPEAN PATENT SPECIFICATION**

(45) Date of publication and mention of the grant of the patent:
**05.08.2020 Bulletin 2020/32**

(21) Application number: **14741219.1**

(22) Date of filing: **26.06.2014**

(51) Int Cl.:
*G01N 33/50* (2006.01)       *G05B 17/02* (2006.01)
*G05B 13/04* (2006.01)       *C12M 1/36* (2006.01)
*C12M 1/34* (2006.01)

(86) International application number:
**PCT/EP2014/063625**

(87) International publication number:
**WO 2014/207166 (31.12.2014 Gazette 2014/53)**

(54) **A METHOD OF DETERMINING OR PREDICTING A CHARACTERISTIC OF A CELL**

VERFAHREN MIT HOHEM DURCHLAUF ZUM SCHNELLEN VORHERSAGEN DER RELATIVEN ZULAUFLEISTUNG EINER TAFEL VON PER KLONUNG VON EINER WIRTSZELLENPOPULATION ABGELEITETEN ZELLEN

PROCÉDÉ RAPIDE ET À RENDEMENT ÉLEVÉ DE PRÉDICTION DE PERFORMANCES D'ÉCOULEMENT CONTINU D'UN PANNEAU DE CELLULES DÉRIVÉES PAR CLONAGE À PARTIR D'UNE POPULATION DE CELLULES HÔTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2013 EP 13173871**
**25.09.2013 EP 13185979**

(43) Date of publication of application:
**04.05.2016 Bulletin 2016/18**

(73) Proprietor: **Valitacell Limited**
**Blackrock, Co. Dublin (IE)**

(72) Inventors:
• **THOMPSON, Ben**
**Sheffield**
**South Yorkshire S8 8QJ (GB)**
• **JAMES, David**
**Sheffield**
**South Yorkshire S11 9LN (GB)**
• **DAVIES, Sarah Louise**
**Stockport**
**South Yorkshire SK2 7DJ (GB)**

(74) Representative: **Purdylucey Intellectual Property**
**6-7 Harcourt Terrace**
**D02 FH73 Dublin 2 (IE)**

(56) References cited:
**WO-A1-97/23642**       **WO-A1-2008/141317**
**CN-A- 101 186 880**       **CN-A- 102 220 239**
**CN-U- 201 569 914**       **US-A1- 2005 204 418**
**US-A1- 2009 117 647**       **US-A1- 2009 287 320**
**US-A1- 2012 190 073**

• **RACHEL LEGMANN ET AL: "A Strategy for clone selection under different production conditions", BIOTECHNOLOGY PROGRESS, vol. 27, no. 3, 29 May 2011 (2011-05-29), pages 757-765, XP055084189, ISSN: 8756-7938, DOI: 10.1002/btpr.577**
• **N. NAKATSU ET AL: "Evaluation of Action Mechanisms of Toxic Chemicals Using JFCR39, a Panel of Human Cancer Cell Lines", MOLECULAR PHARMACOLOGY (THE AMERICAN SOCIETY FOR PHARMACOLOGY AND EXPERIMENTAL THERAPEUTICS), vol. 72, no. 5, 3 August 2007 (2007-08-03) , pages 1171-1180, XP055101018, Bethesda, MD USA ISSN: 0026-895X, DOI: 10.1124/mol.107.038836**
• **THOMAS JOSTOCK ET AL: "Combination of the 2A/furin technology with an animal component free cell line development platform process", APPLIED MICROBIOLOGY AND BIOTECHNOLOGY, SPRINGER, BERLIN, DE, vol. 87, no. 4, 12 May 2010 (2010-05-12), pages 1517-1524, XP019841623, ISSN: 1432-0614**

- RUBEN GODOY-SILVA ET AL: "Physiological responses of CHO cells to repetitive hydrodynamic stress", BIOTECHNOLOGY AND BIOENGINEERING, vol. 103, no. 6, 15 August 2009 (2009-08-15), pages 1103-1117, XP055084184, ISSN: 0006-3592, DOI: 10.1002/bit.22339
- DA CRUZ MELEIRO L A ET AL: "Non-Linear Multivariable Predictive Control of an Alcoholic Fermentation Process Using Functional Link Networks", BRAZILIAN ARCHIVES OF BIOLOGY AND TECHNOLOGY, INSTITUTO DE TECNOLOGIA DO PARANA, BR, vol. 48, no. Special, June 2005 (2005-06), pages 7-18, XP002490882, ISSN: 1516-8913
- Anieta M. Sieuwerts ET AL: "The MTT Tetrazolium Salt Assay Scrutinized: How to Use this Assay Reliably to Measure Metabolie Activity of Cell Cultures in vitro for the Assessment of Growth Characteristics, IC50-Values and Cell Survival", CLINICAL CHEMISTRY AND LABORATORY MEDICINE, vol. 33, no. 11, 1 January 1995 (1995-01-01), XP055376271, DE ISSN: 1434-6621, DOI: 10.1515/cclm.1995.33.11.813

**Description**

Introduction

The invention relates to a method of determining or predicting a characteristic of a cell. In particular, the invention relates to a method of determining the identity of a cell or predicting the fed batch performance of a cell.

Background to the Invention

Many biopharmaceuticals are produced by genetically modified single cell organisms, known as producer cells. A well established system has been established for producing biopharmaceuticals using mammalian producer cells. The first step involves deriving a high-producing cell line that is suitable for large scale production, a process that involves first transfecting, cloning and then characterising a large number of cell lines. This is an expensive and labour and time intensive process, lasting up to 12 months. Once a gene of interest has been transfected and selection pressure applied using the expression system of choice, the next step involves selection and isolation of cells with acceptable growth and recombinant protein production rate. Cells are cultured and expanded to give clonal populations and once typically hundreds of clonal polulations are established, cell line characterisation takes place. Use of so many clonally derived cells necessitates that the each clone is stored separately and documented so that it can be easily identified prior to subsequent use. Currently storage containers for clones are marked ensuring that a clone can be correctly identified. However, if a problem occurs during cataloging the clones, and one or more clone containers is incorrectly labelled, it will not be possible to determine the identity of that clone. Other than sequncing (which is expensive and takes a substantial amount of time), there is no other way of validating the identity of a cryovial other than by trusting the paper work/label.

One step in the process of cell line characterisation is determining the fed batch performance of each clone, which involves monitoring each clone in culture over a period of generally greater than 10 days to determine some fed batch performance metric such as the product yield and IVCD50% for each clone in the panel being tested, which is defined as the integral of viable cell density at the point in culture where the average cellular viability drops below 50%. This is a time and labour intensive process.

Rachel Legmann et al. "A Strategy for clone selection under different production conditions", Biotechnology Progress, vol. 27, no. 3, 29 May 2011 (2011-05-29), pages 757-765, XP-055084189 describes the use of cell growth substrates to identify optimal process conditions in bioreactors while N. Nakatsu et al. "Evaluation of Action Mechansims of Toxic Chemicals Using JFCR39, a Panel of Human Cancer Cell Lines", Molecular Pharmacology, (The American Society for Pharmacology and Experimental Therapeutics), vol. 72, no. 5, 3 August 2007 (2007-08-03), pages 1171-1180, describes methods for predicting the molecular targets or evaluating the action mechanisms of test compounds.

It is an object of the invention to overcome at least one of the above-referenced problems.

Statements of Invention

The invention is based on the finding that the growth response of a cell to a plurality of individual cell stressor molecules can act as a fingerprint or signature, i.e. a stress response fingerprint, that is unique to the cell and can be used as a means of determining or predicting a characteristic of the cell, for example the identity of the cell, the genetic or functional stability of a clonally derived cell, the predicted growth performance of the cell in fed batch culture (predicted fed batch performance), or other characteristics. The cell may be any type of cell, for example a clonally-derived cell or a cell from non-clonal source. In one application of the invention, a panel of cells (clonal or non-clonal) may be processed by incubating each cell with a plurality of cell stressor molecules, measuring the growth response of each cell to each cell stressor, and generating a unique signature or fingerprint for each cell comprising the plurality of growth responses. A panel of such signatures or fingerprints (hereafter "reference cell-specific growth response fingerprints") may be stored and subsequently used as a means of identifying an unidentified cell from the panel, or to confirm the identity of a previously identified cell as an additional checkpoint in quality control. To identify, or confirm the identity, of a cell, it is incubated with a plurality of the same cell stressor molecules, and a query cell specific growth response fingerprint is obtained for the cell. The query fingerprint may then be compared with the panel of reference cell specific growth response fingerprints to identify, or confirm the identity of the cell. In another application, where the reference cell specific growth response fingerprints are obtained from cells with known characteristics, for example cells that are known to perform well or poorly in fed batch culture, comparison of the growth response fingerprint of the query cell with the reference growth response fingerprints allows the fed batch performance, both in terms of growth and productivity, of the query cell to be predicted.

In a first aspect, the invention provides a method of determining or predicting identity of a query cell comprising the steps of:

- simultaneously incubating the query cell with at least three chemical stressor molecules individually in a well of a microtitre plate for 1 to 4 days, in which each chemical cell stressor molecule is provided at a concentration of 0.5 to 2.0 $IC_{50}$;
- determining the growth response of the cell in the presence of each of the at least three chemical stressor molecules to generate a query cell-specific growth response fingerprint;
- comparing the query cell-specific growth response fingerprint with one or more reference cell-specific growth response fingerprints corresponding to one or more cells having known identity; and
- determining or predicting identity of the cell based on the level of correlation between the query cell-specific growth response fingerprint and the one or more reference cell-specific growth response fingerprints.

Suitably, the characteristic of the cell is selected from cell identity, fed batch performance or genetic or functional stability. Thus, the method of the invention suitably relates to determining the identity of a cell, predicting fed batch performance of a cell, predicting relative fed batch performance of a panel of a panel of clonal cells, ideally a panel of clonal cells derived from a single parental host cell population, predicting or determining the genetic or functional stability of the cell.

Although not in accordance with the present invention there is described a method of identifying a cell comprising the steps of:

- incubating the cell with a plurality of chemical cell stressors;
- determining the growth response of the cell in the presence of each of the plurality of chemical cell stressors to generate a query cell-specific growth response fingerprint;
- comparing the query cell-specific growth response fingerprint with one or more reference cell-specific growth response fingerprints corresponding to one or more known cells to identify the cell; and
- when the query cell-specific growth response fingerprint matches or correlates with a reference cell-specific growth response fingerprint, identifying the query cell.

Although not in accordance with the present invention there is described a method for identifying a query clonal cell, the method comprising the steps of:

- incubating the query clonal cell with a plurality of chemical cell stressors;
- determining the growth response of the query clonal cell in the presence of each of the plurality of chemical cell stressors to generate a query clonal cell-specific growth response fingerprint;
- comparing the query clonal cell-specific growth response fingerprint with a plurality of reference cell-specific growth response fingerprints corresponding to a panel of clonal cells.

In a second aspect, the invention relates to a method of generating a reference growth response fingerprint corresponding to a specific cell, comprising the steps of:

- simultaneously incubating the cell with at least three individual chemical cell stressor molecules in wells of a microtitre plate for 1 to 4 days at a concentration of 0.5 to 2 x $IC_{50}$; and
- determining the growth response of the cell in the presence of each of the at least three chemical cell stressor molecules to generate a reference growth response fingerprint.

In a third aspect, the invention relates to a method of generating a panel of reference growth response fingerprints corresponding to a panel of distinct cells, comprising the steps of:

- generating a reference growth response fingerprint for each cell in the panel of cells as hereinbefore defined; and
- storing the panel of reference growth response fingerprints.

In a fourth aspect, the invention provides a system suitable for identifying a cell according to the method hereinbefore defined, the system comprising:

- a microtiter plate comprising a plurality of wells;
- at least three individual chemical cell stressor molecules disposed individually within the wells of the microtiter plate;
- a determination system for determining the growth response of the cell in the presence of each of the three chemical cell stressor molecules provided at a concentration of 0.5 to 2 x $IC_{50}$;
- a storage system for storing a cell-specific growth response fingerprint corresponding to the at least three growth responses of the cell;

- a comparison system configured to compare the cell specific growth response fingerprint with one or more reference cell-specific growth response fingerprints in which the comparison system comprises a computational model configured to input a growth response fingerprint from a query cell, compare the growth response fingerprint with one or more reference growth response fingerprints, and output a content based in part of the comparison result; and
- a display system for displaying an output of the comparison step.

In another embodiment, the invention provides a rapid, high-throughput computer implemented method of predicting relative fed batch performance of a panel of clonal cells derived from a single parental host cell population, the method comprising the steps of:

- simultaneously assaying a level of growth of each clone in the presence and absence of at least three chemical cell stressor molecules individually in wells of a microtitre plate for 1 to 4 days, in which each chemical cell stressor molecule is provided at a concentration of 0.5 to 2.0 $IC_{50}$;;
- comparing the level of growth of each clone in the presence and absence of each of the three chemical cell stressor molecules to provide a normalised growth response value for each clone in each of the three stressed microenvironments;
- inputting a clone-specific growth response fingerprint comprising the normalised growth response value for each clone in each of the stressed microenvironments into a computational model, in which the computational model is generated from growth response fingerprints obtained from a calibration set of clones with known fed batch performance, wherein the computational model is configured to output the predicted fed batch performance value for each clone to predict the relative fed batch performance of the panel of clonal cells.

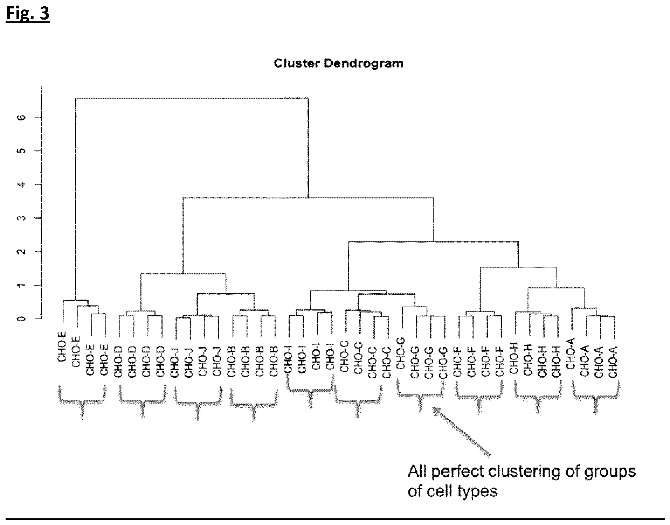

When a batch of new clones are generated from a parental cell line, rather than all or a select few being subjected to expensive and time-consuming growth studies in bioreactors, the method of the invention allows a large number of clones to be rapidly assayed for, and ranked according to, predicted fed batch performance in a rapid and high-throughput manner. This informs on which clones to take forward to scale-up/mini-bioreactor studies, for example only the good clones as illustrated in Fig. 3 below.

The method of this aspect of the invention employs data (cell specific growth response fingerprints) from a panel of pre-validated clones (clones of known fed batch performance), and a computational model, ideally a multiple linear computational model, based on this data, to allow prediction of relative fed batch performance of a new set of clones based on their rapidly obtained chemical fingerprints.

Preferably, the method comprises an aditional step of ranking the clones according to predicted fed batch performance value.

The methods of the invention are typically carried out using a microtitre plate (multiwell plate), in which each assay is performed in a well of a microtitre plate. Suitably, the growth of each cell is assayed in the well of a multiwell plate.

Typically, the assay involves mixing a sample of a clone with a chemical cell stressor, and incubating the mixture from 1 to 4 days, and assaying the level of growth of the cells. Suitably, the clone sample is provided at a concentration of from 0.1 to 1.0 x $10^6$ cells per ml of mixture. Typically, the bioreactor-relevant chemical cell stressor is provided at a concentration of 0.5 to 2 x IC50, ideally about 1 x IC50.

Suitably, the growth of each clone is assayed after a period of incubation of less than 5 days. Ideally, the growth of each clone is assayed after a period of incubation of between 1-4 days, and ideally after 2 and 3 days.

Preferably, the growth of each clone is assayed simultaneously.

The chemical cell stressors are chemicals that cause a reduction in cell growth via one or more of multiple cellular pathways. Suitably, the plurality of chemical cell stressors comprise at least 3, 4, 5, 6, 7, 8, 9, 10, 12, 14, 16, 18, 20, 21, 22, 23, 24, or 25 stressors. Typically, the plurality of chemical cell stressors are selected from the group consisting of amino acid transport inhibitors, cell cycle inhibitors, a source of carbon, a source of osmotic stress, a source of oxidative stress, an inducer of apoptosis, metabolic effectors, a pH modifier, an inhibitor of glycolysis, and a toxin. Typically, the plurality of chemical cell stressors include stressors selected from at least 4, 5, 6 or 7 of the groups consisting of amino acid transport inhibitors, cell cycle inhibitors, a source of carbon, a source of osmotic stress, a source of oxidative stress, an inducer of apoptosis, metabolic effectors, a pH modifier, an inhibitor of glycolysis, and a toxin.

There is described a microtitre plate suitable for generating a growth response fingerprint of a cell and comprising at least 24, 48 or 96 wells, and a plurality of chemical cell stressors disposed individually in at least some of the wells. Typically, the plate comprises at least 6, 7, 8, 9, 10, 12, 14, 16, 18, 20, 21, 22, 23, 24, or 25 chemical cell stressors. Suitably, each chemical cell stressor is disposed in at least two or three wells of the plate (i.e. duplicate or triplicate).

Preferably, the plurality of chemical cell stressors are selected from the group of 2-aminobicyclo-(2,2,1)heptane-carboxylic acid (BCH), D-phenylalanine, $\alpha$-(methylamino)isobutyric acid (MeAIB), Sodium Butyrate (NaBu), cycloheximide, ammonium chloride, Cadmium acetate dihydrate, Cobalt chloride (CoC12), Sodium Chloride (NaCl), Sodium lactate (Na Lac), Aminotriazole (AMT), Menadione Sodium Bisulphite (MSB), Buthionine Sulfoximine (BSO), Mercaptosuccinic Acid (MS), 2,4,Dinitrophenol (24DNP), Sodium Oxamate, 2-deoxyglucose (2dg), 3-bromopyruvate (3-BrPA), Dichloroacetate (DCA), 6-diazo-5-oxo-1-norleucine (1-don), Valproic acid (Val), Sodium Orthovandate (NaV), citric acid, FK866, lactic acid.

There is also described a microtitre plate comprising at least 24, 48 or 96 wells, and a plurality of chemical cell stressors disposed individually in at least some of the wells, in which the plurality of chemical cell stressors comprise at least one chemical cell stressor selected from each of the groups consisting of amino acid transport inhibitors, cell cycle inhibitors, a source of carbon, a source of osmotic stress, a source of oxidative stress, an inducer of apoptosis, metabolic effectors, a pH modifier, an inhibitor of glycolysis, and a toxin.

The term "microtitre plate" or "multiwell plate" as used herein should be understood to mean plates have a plurality of reaction wells, generally at least 12, 24, 48 or 96 wells, each well generally having have a volume of less that 5, 4, 3, 2 or 1ml. The term should be understood to include rigid plates, and plates provided in the form of a flexible roll.

Although not in accordance with the present invention there is additionally described a microtitre plate comprising at least 96 wells, and at least 23 chemical cell stressors disposed individually in wells of the plate, in which the chemical cell stressors consist essentially of 2-aminobicyclo-(2,2,1)heptane-carboxylic acid (BCH), D-phenylalanine, $\alpha$-(methyl-amino)isobutyric acid (MeAIB), Sodium Butyrate (NaBu), cycloheximide, ammonium chloride, Cadmium acetate dihydrate, Cobalt chloride (CoC12), Sodium Chloride (NaCl), Sodium lactate (Na Lac), Aminotriazole (AMT), Menadione Sodium Bisulphite (MSB), Buthionine Sulfoximine (BSO), Mercaptosuccinic Acid (MS), 2,4,Dinitrophenol (24DNP), Sodium Oxamate, 2-deoxyglucose (2dg), 3-bromopyruvate (3-BrPA), Dichloroacetate (DCA), 6-diazo-5-oxo-1-norleucine (1-don), Valproic acid (Val), Sodium Orthovandate (NaV), citric acid, FK866, lactic acid.

There is also described a kit suitable for performing a method of the invention and comprising (a) a microtitre plate of the invention (b) a microtitre plate reader, and (c) a computer program comprising program instructions for (i) comparing the level of growth of each clone in the presence and absence of each of the plurality of chemical cell stressors to provide a normalised growth response value for each clone in each of a plurality of stressed microenvironments, (ii) inputting a clone-specific growth response fingerprint comprising the normalised growth response value for each clone in each of the stressed microenvironments into a computational model, in which the computational model is generated from growth response fingerprints obtained from a calibration set of clones with known fed batch performance, wherein the computational model is configured to output the predicted fed batch performance value for each clone to predict the relative fed batch performance of the panel of clonal cells, and (iii) outputting the predicted fed batch performance value for each clone to predict the relative fed batch performance of the panel of clonal cells.

The invention also provides a computer implemented system configured to predict a relative fed batch performance of a panel of clonal cells derived from a single parental host cell population, the system comprising:

- a determination system for assaying a level of growth of each clone in the presence and absence of a plurality of individual chemical cell stressors provided at a concentration of 0.5 to 2.0 $IC_{50}$ to provide a normalised growth response value for each clone in a plurality of stressed microenvironments; and
- a computational model adapted to process a clone-specific growth response fingerprint comprising the normalised growth response value for each clone in each of the stressed microenvironments, in which the computational model is generated from growth response fingerprints obtained from a calibration set of clones with known fed batch performance, wherein the computational model is configured to output the predicted fed batch performance value for each clone to predict the relative fed batch performance of the panel of clonal cells, and
- means for outputting the predicted fed batch performance value for each clone to predict the relative fed batch performance of the panel of clonal cells.

Typically, the determination system comprises a plate reader, typically a plate reader configured to simultaneously detect changes in light emitted from the wells of a microtitre plate, and suitably correlate the emitted light with level of growth.

Suitably, the computational model is a multiple linear computational model. Ideally, the multiple linear computational model suitably employs a least squares solution to fit parameters (i.e. levels of cell specific growth responses) to the observed fed batch performance (preferably defined as IVCD50).

There is also described a computer program which when executed on a computer causes the computer to perform a method of predicting relative fed batch performance of a panel of clonal cells derived from a single cell line according to the invention.

There is also described a computer program recording medium storing a computer program according to the invention.

In this specification, the term "cell" refers to any cell type, including prokaryotic or eukaryotic cells. Suitably, the cell is a eukaryotic cell, ideally a mammalian cell. Typically, the cell is a producer cell, preferably a mammalian producer cell. The cell may be clonally-derived or non-clonal. In a preferred embodiment, the cell is a clone from a panel of clonal cells, derived from a single parental cell population or derived from different transfected cells. The cell may also be from a distinct cell line, for example a genetically modified cell (i.e. cells with "knock-out" or "knock-in" mutations. The cell line may also be derived by directed evolution, by selection of cells which have adapted to a specific environment of interest. The term "panel of distinct cells" should be understood to mean a panel of cells that are different from each other. For example, the panel of cell may comprise a panel of clones that are all derived from a single parental cell population. Alternatively, the panel of cells may comprise cells of independent clonal origin, for example cells carrying different transgenes or different mutations, of the panel of cells may comprise non-clonal cells. The cell line may also be derived by directed evolution, by selection of cells which have adapted to a specific environment of interest. The panel of cells may also comprise cells of different cell type, for example different cell lines, different strains of bacteria or fungi, cells of the same cell type but at a different stage of development, and cells of the same cell type that differ genetically, for example cells of the same type or origin or cells derived from the same parental cell population that carry different transgenes.

In this specification, the term "panel of clonal cells" should be understood to mean a panel of clonal cell populations derived from a single cell line, and comprising from 2 to 500 or more clonal cell populations. Methods for generating panels of clonal cells are well known to a person skilled in the art, and described in Production of recombinant protein therapeutics in cultivated mammalian cells (2004),Wurm, Florian M, New York, NY, Nature Biotechnology 22 (2004), S. 1393-1398. Typically, the panel of clonal cell populations include from 10-500, 20-500, 30-500, 40-500, 50-500, 60-500, 70-500, 80-500, 90-500 or 100-500 clonal cell populations. Typically, the panel of clonal cell populations include from 100-500, 100-400, 150-400, 150-350 clonal cell populations.

In this specification, the term "identifying a cell" should be understood to mean identification of an unknown cell, or confirming the identity of a cell of known or suspected identity.

In this specification, the term "fed batch performance" should be understood to mean proliferation or production performance of the cell in fed batch culture. Preferably the term means IVCD50%, which is defined as the integral of viable cell density at the point in culture where the average cellular viability drops below 50%.

In this specification, the term "relative fed batch performance of a panel of clonal cells" should be understood to mean the fed batch performance of the clones in the panel relative to one another. Thus, in one embodiment, the clones are stratified according to their fed batch performance. In another embodiment, the clones are stratified to pick out the best performing clones, the worst performing clones, or both.

The invention involves incubating a cell with a plurality of cell stressor molecules. This means that the cell is incubated with each of the cell stressor molecules individually, to obtain a growth response of the cell in the presence of each cell stressor molecule. Incubation of the cell with the cell stressor molecule may be performed in any suitable reaction vessel, for example in the wells of a microtitre plate. Typically, the assay involves mixing a cell sample with a chemical cell stressor, and incubating the mixture from 1 to 4 days, and assaying the level of growth of the cells. Suitably, the cell sample is provided at a concentration of from 0.1 to 1.0 x $10^6$ cells per ml of mixture. Typically, the cell stressor is provided at a concentration of 0.5 to 2 x IC50, preferably about 0.8 to 1.2 x IC50, and ideally about 1 x IC50. Suitably, the growth of each clone is assayed after a period of incubation of less than 5 days. Ideally, the growth of each clone is assayed after a period of incubation of between 1-4 days, and ideally after 2 and 3 days. Preferably, the growth of each clone is assayed simultaneously.

The term "cell stressor molecule" or "cell stressor" should be understood to mean a molecule or compound that causes a reduction in cell growth via one or more of multiple cellular pathways, for example pathways involved in cell growth or metabolism. The stressor molecules are employed on the basis of their ability to simulate bioreactor stresses, for example one or more of oxidative stress, cell cycle inhibition, inhibition of protein production, and inhibition of NAD. Typically, the plurality of chemical cell stressors are selected from the group consisting of amino acid transport inhibitors (i.e. BCH), cell cycle inhibitors (i.e. sodium butyrate), a source of carbon, an inducer of osmotic stress (i.e. sodium lactate), an inducer of oxidative stress (tert-BHP), an inducer of apoptosis (cycloheximide, TNF), a metabolic effector (i.e. a chemical that directly modulates metabolism), a pH modifier (i.e. citric acid), an inhibitor of glycolysis (i.e. 3-bromopyruvate), and a toxin (i.e. a compound or molecule that is toxic to a cell, for example cadmium). Preferably, the plurality of chemical cell stressors are selected from the group consisting of amino acid transport inhibitors, cell cycle inhibitors, an inducer of osmotic stress, an inducer of oxidative stress, an inducer of apoptosis, a metabolic effector, an inhibitor of glycolysis, and a toxin (i.e. a compound or molecule that is toxic to a cell). Typically, the plurality of chemical cell stressors include stressors selected from at least 4, 5, 6 or 7 of the groups consisting of amino acid transport inhibitors, cell cycle inhibitors, a source of carbon, an inducer of osmotic stress, an inducer of oxidative stress, an inducer of apoptosis, a metabolic effectors, a pH modifier, an inhibitor of glycolysis, and a toxin.

Examples of metabolic (catabolism) effectors are 1) chemicals which directly interfere with metabolic enzymes such as 2-deoxyglucose which competitively inhibits hexokinase (the first enzyme in the glycolysis pathway) and 2) chemicals that directly interfere with energy metabolism machinery such as 24DNP which causes pores in the mitochondrial membrane thus dissipating the proton gradient and lowering ATP production. Examples of metabolic (anabolism) effectors are FK866 which inhibits the synthesis of NAD. Additionally cyloheximide inhibits protein synthesis (and causes apoptosis).

In this specification, the term "plurality" as applied to cell stressor molecules should be understood to mean at least three different cell stressor molecules. Typically, the term refers to at least 4, 5, 6, 7, 8, 9, 10, 11, 12, 13, 14, 15, 16, 17, 18, 19 or 20 different cell stressor molecules. Thus, the method of the invention may be performed by employing as few as three distinct cell stressor molecules to generate the cell-specific growth response fingerprint, for example: $CoCl_2$, NAV and MSB; Cadm, MSB and 2DG; and Cadm, dphe, MeiAB.

The term "growth response of the cell" refers to the growth of the cell in the presence of a cell stressor molecule. Typically, the growth response is a normalised growth response, which is determined by measuring the growth of in the presence and absence of the cell stressor molecule and determining the difference in growth response due to the presence of the cell stressor molecule. Growth of cells may be determined using any technique known in the art. In one embodiment, a dye is added to the incubation mixture and a signal emitted by the dye is monitored over time and correlated with growth. For example, a fluorescent or phosphorescent dye may be employed. Examples of suitable dyes are redox dyes, such as Presto blue®. (Invitrogen, Paisley, UK)

The term "cell-specific growth response fingerprint" refers to a plurality of growth responses for a specific cell obtained by reacting the cell with a plurality of cell stressor molecules individually. The fingerprint may be embodied as a plurality of growth response values, or may be embodied in the form of a graph or any other type of visual presentation such as a pattern.

The term "query cell specific growth response fingerprint" refers to a cell specific growth response fingerprint corresponding to an unidentified cell or a cell whose identity is to be confirmed. The term "reference cell specific growth response fingerprint" refers to a growth response fingerprint for a cell the identity of which is known. Generally, the query and reference growth response fingerprints will be generated using the same set of cell stressor molecules.

The term "calibration set of clones" refers to a plurality of clones, each having a growth response fingerprint and a known fed batch performance. Typically, the calibration set of clones includes clones exhibiting a range of fed batch performance abilities from what the end user would consider 'good' performers to what the end user would consider 'bad' performers. Suitably, the calibration set of clones comprises at least 3, 4, 5, 6, 7, 8, 9, 10, 12, 14, 16, 18, 20, 22, 24, or 26 clones.

The term "stressed microenvironment" should be understood to mean an environment that includes a chemical cell stressor. Generally, this means that a clone is assayed for growth in a well of microtitre plate in the presence of the chemical cell stressor.

In this specification, the term "rapid" should be understood to mean a method of predicting fed batch performance that takes less than five days, and ideally less than four or three days.

In this specification, the term "high-throughput" should be understood to mean a method in which a large number of samples, for example 20-500, can be assayed simultaneously. In one embodiment, this involves assaying the growth of the clones in a multiwell plate, for example a 48, 96, or 192 well plate.

In this specification, the term "computational model" typically refers to a multiple linear computational model. Ideally, the multiple linear computational model employs a least squares solution to fit parameters (i.e. levels of cell specific growth responses) to the observed fed batch performance (preferably defined as IVCD50).

One aspect of the invention involves comparing a query cell specific growth response fingerprint with one or more reference cell specific growth response fingerprints to identify a match or level of correlation between the query and reference growth response fingerprint(s). Various methods may be employed to match a query fingerprint with one of the reference fingerprints including mathematical modelling, pattern recognition, or by visual inspection. For example, the growth responses for an unidentified cell may be plotted against cell stressors to provide a graph representing the growth response fingerprint for the cell, and the growth response fingerprint may be compared visually with the reference growth response fingerprints provided in the same format to identify a matching reference growth response fingerprint and thereby identify the cell. In a preferred embodiment, the comparison step may be performed by mathematical modelling using 'Linear discriminant analysis' and 'nearest neighbour Euclidian distance minimisation', using a subset of the chemical growth responses. Typically, the model employs a 'within groups' leave one out method. With this, 'training data' is optionally made by leaving one out for each group (i.e. 10 samples) and then building an LDA model, and then predicting the cell type for each of the 10 distinct cell types which were left out. Other methods of matching or correlating a query fingerprint with one or more reference fingerprint(s) involves simple Euclidian matching or hierarchical cluster analysis.

There is also described a system or kit for identifying a cell. The system or kit typically comprises a device having a plurality of reaction chambers. Preferably, the device is a microtitre plate, typically a microtitre plate having at least 12, 24, 48, or 96 wells.

The system or kit typically comprises a plurality of cell stressor molecules. Preferably, the cell stressor molecules are disposed individually within the wells of the microtitre plate, and are preferably adhered to the wells of the microtitre plate.

The system or kit comprises a determination system for determining the growth response of the cell in the presence of each of the plurality of chemical cell stressors. Typically, the determination system comprises a microtitre plate reader configured to detect growth of cells in the wells of the microtitre plate. Suitable microtitre plate readers are commercially available, and are sold by the company BMG. The determination system typically has computer executable instructions to provide e.g., growth response data in computer readable form.

The system or kit also comprises a storage system and a comparison system. These functional modules can be executed on one, or multiple, computers, or by using one, or multiple, computer networks. The determination system has computer executable instructions to provide e.g., sequence information in computer readable form.

The information determined in the determination system can be read by the storage system. As used herein the "storage system" is intended to include any suitable computing or processing apparatus or other device configured or adapted for storing data or information. Examples of an electronic apparatus suitable for use with the present invention include a stand-alone computing apparatus, data telecommunications networks, including local area networks (LAN), wide area networks (WAN), Internet, Intranet, and Extranet, and local and distributed computer processing systems. Storage devices also include, but are not limited to: magnetic storage media, such as floppy discs, hard disc storage media, magnetic tape, optical storage media such as CD-ROM, DVD, electronic storage media such as RAM, ROM, EPROM, EEPROM and the like, general hard disks and hybrids of these categories such as magnetic/optical storage media. The storage system is adapted or configured for having recorded thereon growth response information and growth response fingerprint information. Such information may be provided in digital form that can be transmitted and read electronically, e.g., via the Internet, on diskette, via USB (universal serial bus) or via any other suitable mode of communication.

The storage system may have reference growth response fingerprint information stored thereon. As used herein, "stored" refers to a process for encoding information on the storage device. In one embodiment the reference data stored in the storage device to be read by the comparison module is compared, e.g., comparison of a query cell-specific growth response fingerprint with a set or panel of reference growth response fingerprints.

The "comparison system" can use a variety of available software programs and formats for the comparison operative to compare query growth response fingerprint with reference growth response fingerprints and identify a "match". In one embodiment, the comparison module is configured to use pattern recognition techniques to compare information from one or more entries to one or more reference data patterns. The comparison module may be configured using existing commercially-available or freely-available software for comparing patterns, and may be optimized for particular data comparisons that are conducted. The comparison module provides computer readable information related to the genotype of the sample. Preferably, the comparison system employs a computational model for comparison purposes.

The comparison module, or any other module of the invention, may include an operating system (e.g., UNIX) on which runs a relational database management system, a World Wide Web application, and a World Wide Web server. World Wide Web application includes the executable code necessary for generation of database language statements (e.g., Structured Query Language (SQL) statements). Generally, the executables will include embedded SQL statements. In addition, the World Wide Web application may include a configuration file which contains pointers and addresses to the various software entities that comprise the server as well as the various external and internal databases which must be accessed to service user requests. The Configuration file also directs requests for server resources to the appropriate hardware--as may be necessary should the server be distributed over two or more separate computers. In one embodiment, the World Wide Web server supports a TCP/IP protocol. Local networks such as this are sometimes referred to as "Intranets." An advantage of such Intranets is that they allow easy communication with public domain databases residing on the World Wide Web (e.g., the GenBank or Swiss Pro World Wide Web site). Thus, in a particular preferred embodiment of the present invention, users can directly access data (via Hypertext links for example) residing on Internet databases using a HTML interface provided by Web browsers and Web servers.

The comparison module typically provides a computer readable comparison result that can be processed in computer readable form by predefined criteria, or criteria defined by a user, to provide a content based in part on the comparison result that may be stored and output as requested by a user using a display system.

In one embodiment of the invention, the content based on the comparison result is displayed on a computer monitor. In one embodiment of the invention, the content based on the comparison result is displayed through printable media. The display module can be any suitable device configured to receive from a computer and display computer readable information to a user. Non-limiting examples include, for example, general-purpose computers such as those based on Intel PENTIUM-type processor, Motorola PowerPC, Sun UltraSPARC, Hewlett-Packard PA-RISC processors, any of a variety of processors available from Advanced Micro Devices (AMD) of Sunnyvale, California, or any other type of processor, visual display devices such as flat panel displays, cathode ray tubes and the like, as well as computer printers of various types.

In one embodiment, a World Wide Web browser is used for providing a user interface for display of the content based on the comparison result. It should be understood that other modules of the invention can be adapted to have a web browser interface. Through the Web browser, a user may construct requests for retrieving data from the comparison module. Thus, the user will typically point and click to user interface elements such as buttons, pull down menus, scroll bars and the like conventionally employed in graphical user interfaces.

There is also described a method of identifying a cell comprising the steps of preparing a growth response fingerprint for the cell, in which the growth response fingerprint comprises the growth response of the cell to a plurality of chemical cell stressor molecules, comparing the growth response fingerprint for the cell with a plurality of growth response fingerprints corresponding to cells of known identity, and identifying the cell when the growth response fingerprint for the cell matches one of the growth response fingerprints corresponding to the known cells. Typically, the comparison step employs mathematical modelling.

There is also described a method of identifying a characteristic of a cell comprising the steps of:

- incubating the cell with a plurality of chemical cell stressors;
- determining the growth response of the cell in the presence of each of the plurality of chemical cell stressors to generate a query cell-specific growth response fingerprint;
- comparing the query cell-specific growth response fingerprint with one or more reference cell-specific growth response fingerprints corresponding to one or more cells having known characteristics; and
- identifying a characteristic of the cell based on the level of correlation between the query cell-specific growth response fingerprint and the one or more reference cell-specific growth response fingerprints.

Typically, the step of identifying a characteristic of the query cell comprises a step of matching the query cell-specific growth response fingerprint with a reference cell-specific growth response fingerprint.

The characteristic may be any characteristic of the cell, for example:

- the identity of the cell (where a query fingerprint is compared with reference fingerprints from cells of known identity),
- the growth characteristics of the cell (where a query fingerprint is compared with reference fingerprints from cells of known growth characteristics),
- the predicted fed batch performance of the cell (where a query fingerprint is compared with reference fingerprints from cells of known fed batch performance),
- the ability of a cell (i.e. a mammalian producer cell) to express recombinant protein (where a query fingerprint is compared with reference fingerprints from cells of known producing capacity),
- the ability of the cell to survive and/or grow in specific environments, (where a query fingerprint is compared with reference fingerprints from cells of that are known to survive and/or grow in specific environments),
- the stability (genetic and/or functional) of clonal cell lines (in which a query cell fingerprint is compared with fingerprints from cells that have demonstrated functional and/or genetic stability and/or functional and/or genetic instability over time, during storage, or throughout successive generations of growth). This aspect of the invention allows the identification of genetic or functional instability in clones, which is undesirable for cell lines used in manufacturing.

The level of correlation between the query cell-specific growth response fingerprint and the one or more reference growth response fingerprints may be determined by any means, including visual comparison of data representing the fingerprints (for example, comparing graphs representing the fingerprints) or by means of mathematical modelling to identify, for example, a best fit or closest match. Typically, the mathematical modelling employs linear discriminant analysis (LDA), although other modelling approaches based on "matching things to groups" may be employed.

Brief Description of the Figures

Figure 1: A representation of the 4 'chemical fingerprints' generated from each CHO cell line (A to J).

Figure 2: An example of a chemical growth fingerprint from a clone. Cells were grown for 72 hours in the presence or absence of a chemical of interest in a 96 well plate. Relative levels of cell growth were assayed using the "Presto-Blue" method as described above.

Figure 3: A dendogram displaying the goodness of cell type clustering using a 'best correlation' method and "Ward's Hierarchical Clustering".

Figure 4: A two dimensional example of how a linear combination of chemical responses can be used to maximally separate two different groups.

Figure 5: A two dimensional example of how a linear combination of chemical responses can be used to classify a new data point (triangle point) into a group using the 'maximum likelihood' classification.

**Figure 6:** A two dimensional representation of how PCA can be used to reduce the dimensionality of a data set without losing substantial amounts of information.

**Figure 7:** Data could be expressed as 7 linear combinations of the chemical responses without while still being able to represent the vast majority of the variation in the data.

**Figure 8:** A representation of the ability of the LDA model to predict the CHO cell type of 'unknown' CHO fingerprints with 100% accuracy.

**Figure 9 (2):** An illustration of the fit of the multiple linear model to predict the relative performance ability (Y axis) from the model based on the clone chemical fingerprints.

**Figure 10 (3):** An example of how the multiple linear model can use clone chemical fingerprints to partition the set of clones into 'good' and 'bad' performers thus aiding the process of selecting which clones to take forward to the next step of clone screening.

**Figure 11 (4):** A demonstration, using bootstrap 3 fold cross validation techniques of how the multiple linear model is able to predict data not used to build the model (i.e. blindly predict the relative fed batch performance).

**Figure 12:** An illustration of a computer system configured to perform one or more of the methods of the invention.

Detailed Description of the Invention

Exprimental

*Cell culture (cell identification):*

10 types of clonally and non-clonally derived CHO cell lines were sourced. These were all industrially important cell lines from numerous industrial/commercial sources. These consisted of both clonally derived stable-producing clonal cell lines running on two distinct expression systems, clonally derived non-producing CHO cells, episomal replicating cells, DHFR-ve cells and non-clonally derived cells from different commercial CHO cell parentage. These are referred to as CHO-A, CHO-B, CHO-C, CHO-D, CHO-E, CHO-F, CHO-G CHO-H, CHO-I and CHO-J. These were all grown in their proprietary recommended culture media and culture conditions.

*Cell culture (predicting IVCD50 of panel of clonal cells):*

PM-CHO Cells were culture CD-CHO media (Invitrogen, Paisley, UK), 8mM L-Glutamine (Invitrogen, Paisley, UK), 1% HT supplement (Invitrogen, Paisley, UK) 12.5 $\mu$g/ml puromycin (Invitrogen, Paisley, UK). Alternative (panel 2) CHO cells were cultured in CD-FORTI CHO (Invitrogen, Paisley, UK). Cells were routinely sub-cultured on an alternating 3 / 4 day regime. Using data from fed batch studies (with commercially available feed), IVCD50 (Integral of viable cell density at the point where culture viability drops below 50%) were used as the metric to judge the cell's ability to grow in fed batch culture. These IVCD50s were used to rank the cells. A ranking system was selected as this would be more robust to outliers and skewed data.

*Small scale determination of cell growth:*

Assaying cell growth in 96 well plates: "Presto Blue" (Invitrogen, Paisley, UK) was mixed 1:1 with CD-CHO media and 20 ul of this was added to each well. The plate was machine shaken and subsequently incubated for 30 minutes at 37C in a static humidified incubator. After incubation the fluorescence of the wells was measured (excitation 535nm emission 620 nm) using a fluoroskan ascent (Thermo-Fisher, Loughborough, UK) plate reader. It has been demonstrated prior that fluorescence is linearly correlated with viable cell density in the wells.

*Preparation of the chemical test plate:*

Method: The plurality of cell stressor molecules employed were:

2-aminobicyclo-(2,2,1)heptane-carboxylic acid (BCH)
D-phenylalanine -(D-Phe)
$\alpha$-(methylamino)isobutyric acid (MeAIB)
Sodium Butyrate (NaBu)
Cycloheximide
Ammonium chloride
Cadmium acetate hexhydrate
Cobalt chloride (CoCl$_2$)
Sodium Chloride (NaCl)
Sodium lactate (Na Lac)
Aminotriazole (AMT)
Menadione Sodium Bisulphite (MSB)
Buthionine Sulfoximine (BSO)
Mercaptosuccinic Acid (MS)
2,4,Dinitrophenol (24DNP)
Sodium Oxamate
2-deoxyglucose (2dg)
3-bromopyruvate (3-BrPA)
Dichloroacetate (DCA)
6-diazo-5-oxo-1-norleucine (1-don)
Valproic acid (Val)
Sodium Orthovandate (NaV)
Citric acid
FK866
Lactic acid

Using an 'in house' parental cell line, inhibitory dose 50 (IC50) concentrations of the above chemicals were identified through growth studies. Here IC50 is defined as the concentration of the chemical, in question, which inhibits normal cell growth by 50% over a 3 day period. Once IC50s were established, 96 well plates were set up containing the above selection of chemicals at the IC50 concentration and including control wells which contained only cell growth media.

## EXAMPLE 1: Identification of cell using stress response fingerprint

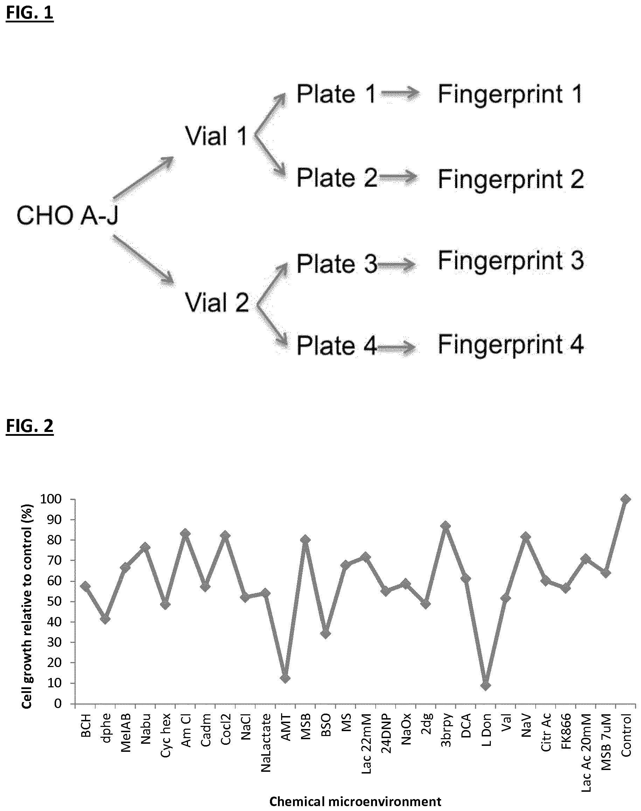

Duplicate vials for each cell line (CHO-A to CHO-J) were revived from cryostasis. These were cultured in their recommended growth media until the growth profile for each vial had become stable (around 4 successive subcultures post thawing). Duplicate vials were cultured separately throughout. Duplicate vials enabled an estimation of 'vial to vial' variation. Each cell line from a duplicate vial was used to seed a chemical plate on day 3 post subculture (Figure 1). Essentially each duplicate vial cell line, was grown in the plate containing the above mentioned chemicals for 3 days. Cells from each duplicate vial were used to seed duplicate plates, e.g. 4 plates per cell line.

After this period the level of cell growth in the plates (i.e. in the presence and absence of chemicals as mentioned above) was measured using the "Presto Blue" assay as described above. This allowed a cell specific growth fingerprint to be identified for each CHO cell line. Here a growth finger print is defined as the level of cell growth in each chemical microenvironment relative to the growth of the cells in the control well (i.e. the wells containing just media.). An example of this is illustrated in Figure 2.

Essentially, two different modelling approaches were employed to use aspects of the cellular chemical fingerprint to identify the "cell type". These two methods, despite their substantially differing approaches, were both able to reliably identify cell type from fingerprint alone. These two approaches were "Euclidatian Distance, Wards method Clustering" and "Linear discriminant analysis"

## Details of ""Euclidatian Distance, Wards method, Clustering" modelling.

This approach seeks to find the best correlation of aspects of a fingerprint (chemical responses) to identify the cell type. Given a query fingerprint, it seeks to find the group of cell types which gives the best correlation and subsequently, allocate the query cell to this group.

The goodness of clustering was seen as thus: A query cell type is only classified as being in a specific group (cell type) if the lowest correlation to any other cell type within that group is higher than the highest correlation to any cell in a different group (other cell types). This gave stringent criteria for evaluation of "which subsets" of the 25 chemical responses gave the best separation of groups of cell types by "minimising Euclidian distance" i.e. correlation. This means maximising the within groups correlations compared to the outside groups correlations

All subsets of the chemicals were examined and it was discovered that numerous combinations of the 25 chemical growth responses could be used to successfully cluster all cell types together. The smallest set of these in this instance was 7 chemical responses, namely, d-phenylalanine, MeiAB, Cycloheximide, AmCl, Cadmium, lactate and 2-deoxyglucose.

A cluster dendogram illustrating the goodness of clustering is included in Figure 3.

*Details of "Linear discriminant" modelling approach.*

For each cell type, e.g. CHO A to J, duplicate fingerprints were obtained for each duplicate 'vial origin' of cell type (Fig 1). The 4 fingerprints generated from each CHO cell type was used to represent that CHO cell type as a 'group'.

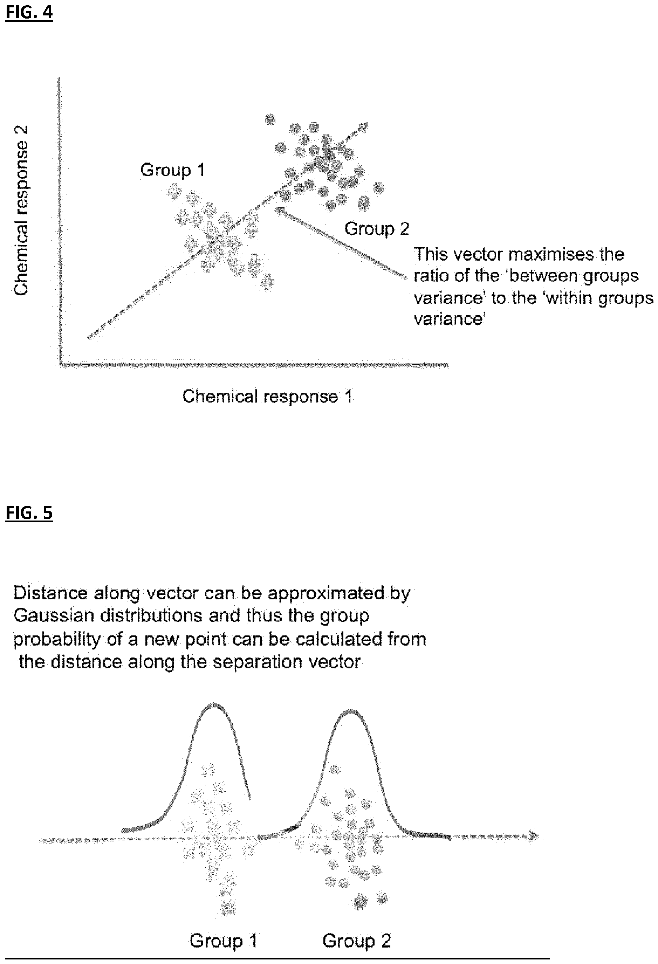

Using these fingerprints, linear discriminant analysis was used to generate a linear combination of distinct aspects of their chemical finger-print that best maximizes the 'between' CHO cell type variation to the 'within' CHO cell type variation (i.e. that between the 4 fingerprints generated for each CHO cell type). A two dimensional hypothetical example of this is illustrated in figure 4. Here, within CHO cell types variation can be seen as the variation of the fingerprints from the same CHO cell type i.e. variation due from the duplicate vials (vial to vial variation) and the duplicate plates (plate to plate variation).

Using information from this linear combinations of chemical responses, this allows the identification of new data points into appropriate groups. For example given the 'chemical fingerprint' of an unknown 'query' CHO cell type, the LDA model can be used to predict which CHO cell type the query is most likely to be and also to put a probability on which CHO cell type group (i.e. CHO A to J) the query belongs to. This is illustrated in figure 5.

*Data dimension reduction to give more predictive LDA analysis*

Due to the large size of the data set, there were many 'multi-colinear' variables (variable whose values can be strongly predicted from linear combinations of other variables). This can lead to poor cross validation, i.e. the ability of the LDA model to predict classify new data points using data which was not used to make to LDA model.

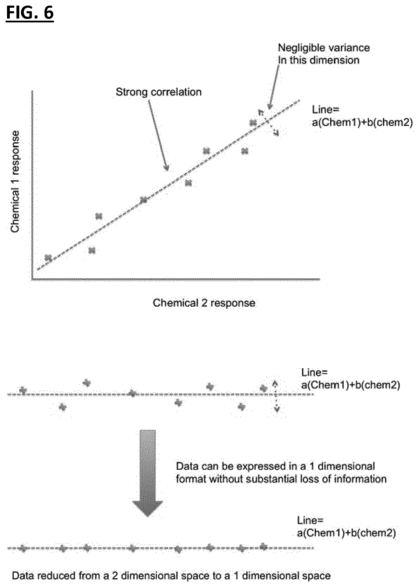

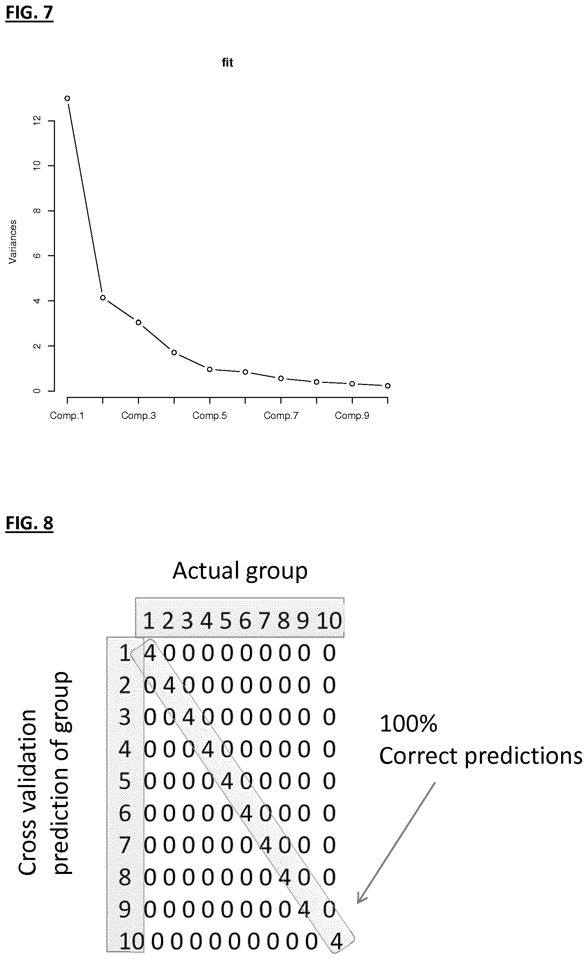

To address this, the size (dimensionality) of the data set was reduced using "principle component analysis". This allows data to be mapped onto a lower dimensional space without losing substantial amounts of information. An illustration of this technique can be seen in Figure 6. This technique was used to reduce the dimensions of the data space from 25 dimensions to 7 dimensions. This is illustrated in Figure 7, which indicates that a vast amount of the variation in the data can be captured using 7 principle components of the data. Using these subsets of these dimensions, the best LDA analysis was performed. In this context, LDA is defined as the best linear combination of principle components which maximises the between groups variation relative to the within groups variation.

The best linear discriminant model found used 4 components of the principle component 'breakdown'. This model was able to identify 'which fingerprint belonged to which CHO cell type' from the 40 different fingerprints generated with 100% accuracy.

*Cross-Validation of the ability of the optimal LDA model to predict the CHO cell type using new, 'unknown' (i.e. unknown to the model) fingerprints.*

Importantly, the optimal LDA model was 'Cross-validated using a "leave one out" (LOO) cross-validation technique. Essentially for each of the 40 fingerprints, 1 would be ignored (seen as 'test data') and an LDA model would be built using the other 39 fingerprints (seen as 'training data'). This model was then used to predict the CHO cell type of the 'test data' fingerprint.

This process is repeated using each of the 40 fingerprints as the 'test' data points, and the CHO cell type (class) of each 'query' was predicted using the LDA model built on the training data set.

*Within groups "Leave one out" cross validation.*

To provide a more stringent cross-validation of the data, a 'within groups leave one out cross validation' method was developed. Essentially, since there were 10 'groups' (CHO cell types A-J), with 4 members in each groups, for a 1 member was ignored from each group (10 ignored in total). Subsequently, the remaining 30 data points (the "training data set") were used to build a best LDA model. Using this, the groups of the 'unknown' (to the model) ignored data points (the "test data set") were predicted. This was repeated until all data points had been used in the test and training data set.

In essence, using "new" data points (test data sets), the LDA model based on these components was able to correctly predict the cell type (CHO A-J) of a cell based on its fingerprint, with 100% accuracy (Figure 8).

The examples above demonstrate that using the plate based 'metabolic fingerprint' methods of the invention, it is possible to confidently identify a specific CHO cell type from a panel of closely related CHO cell types. The methods of the invention is also applicable for the identification of non-CHO types and even in the identification of non-mammalian cells.

**EXAMPLE 2** - **Prediction of fed batch performance (IVCD50) of a panel of clonal cells**

A panel of size 20 of clonal cell lines with known fed batch performance metrics (i.e. IVCD50) was generated in house. Each clonal cell line was grown in the plate containing the above mentioned chemicals for 3 days. After this period the level of cell growth in the plates (i.e. in the presence and absence of chemicals as mentioned above) was measured using the "Presto Blue" assay as described above. This allowed a cell specific growth fingerprint to be identified for each clonal cell line. Here a growth finger print is defined as the level of cell growth in each microenvironment relative to the growth of the cells in the control well (i.e. the wells containing just media.)

An example of a typical growth fingerprint for a clone is shown in Fig. 2.

Using information from these chemical fingerprints, a multiple linear model was built using responses to individual chemicals as parameters.

A multiple linear model is defined as that satisfying the equation.

$$\hat{\beta} = (\mathbf{X}^{\mathrm{T}}\mathbf{X})^{-1}\mathbf{X}^{\mathrm{T}}y = \left(\frac{1}{n}\sum \mathbf{x}_i \mathbf{x}_i^{\mathrm{T}}\right)^{-1}\left(\frac{1}{n}\sum \mathbf{x}_i y_i\right).$$

Where X represents the matrix containing appropriate data from the explanatory variables and Y is the matrix of dependent (or response) variables. This is essentially finding a least squares solution to fit parameters (i.e. levels of cell specific growth responses) to the observed fed batch performance (here defined as IVCD50)

An example of a multiple linear model built from the plurality of clone microenvironments is

$$Y = -0.978(\text{Sodium Butyrate response}) + 0.763(\text{Cadmium response}) + 0.364(\text{Mercaptosuccinic acid response}) + 2$$

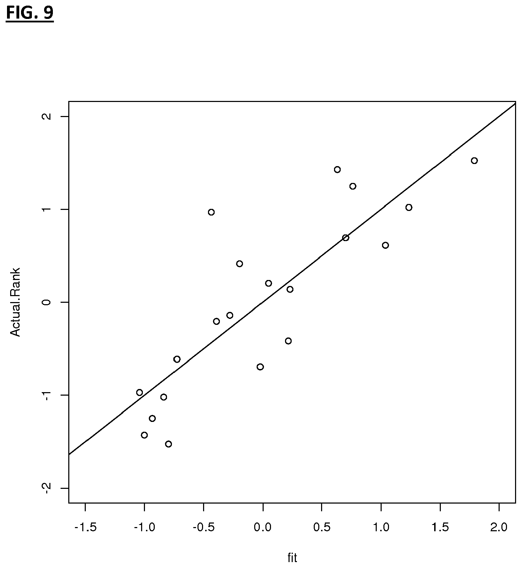

Where Y is the predicted standardized rank (which can easily be interpreted as rank). A graph illustrating the goodness of model fit using the above parameters is shown in Fig. 9. The $R^2$ value for this model is 0.7. This can be interpreted that 70% of the behaviour of the data can be explained by the model. Further use of this model can be illustrated as its ability to differentiate between the top and bottom 50% of clones in terms of their rank.

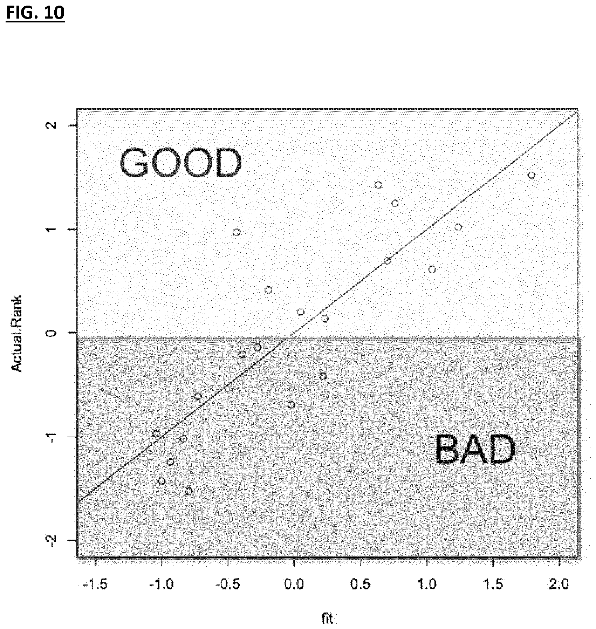

With the above mentioned modelling approach this can predict whether a clone is in the top 50% (good) or the bottom 50% (bad) with 85% accuracy as illustrated in Fig. 10.

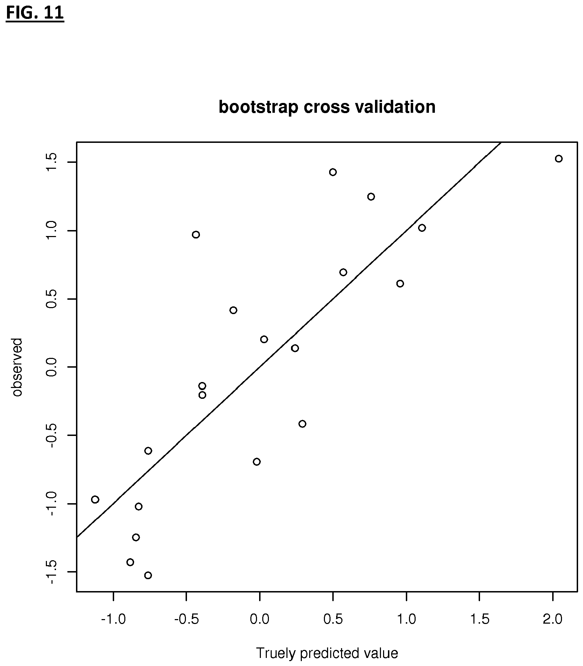

By using bootstrap cross validation techniques it can be shown that a model built in the above way can be predictive of future data, i.e. data not used in the model. Fig. 11 illustrates the predictive ability of the modelling approach to build predictive models. The $R^2$ value for the bootstrap cross-validation is 0.66 which indicated the ability to predict new data.

This model, build on a set of clones with known fed batch performance can use the 'fingerprint' of new clones and be able to predict their relative fed batch performance. Initially a model is generated using a panel of clones with known fed batch performance (in the above example 20 were used but there is no limitation to how many can be used, and as few as three may be employed - it is preferable to use at least 20 clones of known performance to build the model). Subsequently when a large panel of clones is derived in the process of cell line development, these will be "screened" in the multiple microenvironment plates, and using the model generated from clones with known performance characteristics, it is possible to rank these clones or at least give information as to the likelihood of their future fed batch performance capabilities therefore aiding the process of selecting which clones are taken forward to the next stage of clones screening (i.e. small scale fed batch studies).

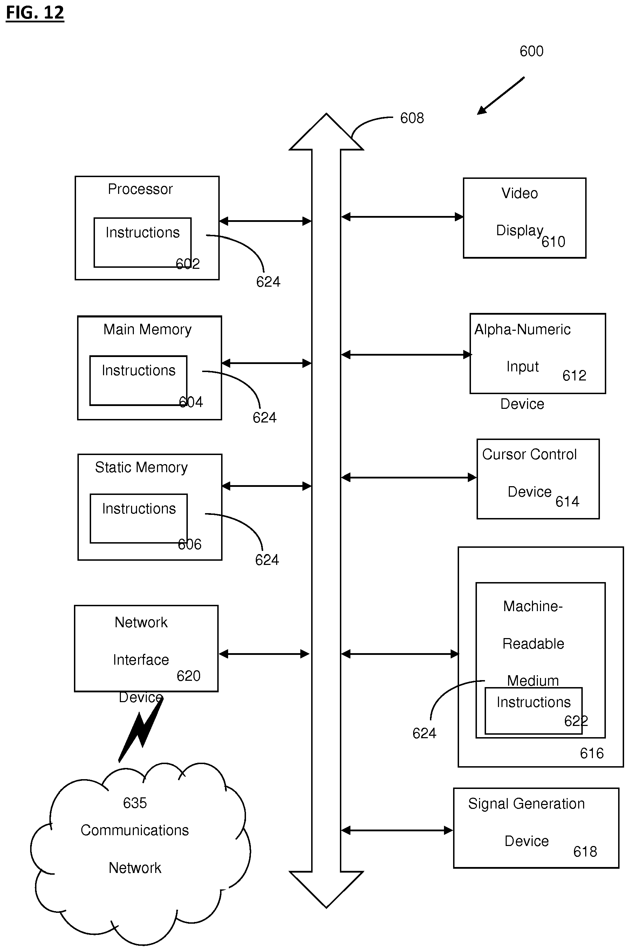

Additionally, referring now also to Figure 12, at least a portion of the systems, methodologies and techniques described with respect to the exemplary embodiments of present disclosure can incorporate a machine, such as, but not limited to, computer system 600, or any other computing device within which a set of instructions, when executed, may cause the machine to perform any one or more of the methodologies or functions discussed above. The machine may be configured to facilitate various operations conducted by the systems disclosed herein. For example, the machine may be configured to, but is not limited to, assist the systems by providing processing power to assist with processing for the computer implemented system configured to predict a relative fed batch performance of a panel of clonal cells

derived from a single parental host cell population in a well, by providing storage capacity for storing instructions or data traversing the systems, or by assisting with any other operations conducted by or within the systems.

In some embodiments, the machine operates as a standalone device. In some embodiments, the machine may be connected (e.g., communications network 635) to and assist with operations performed by other machines. The machine may be connected with any component in the system, for example a microplate reader configured to generate a growth response profile for a cell. In a networked deployment, the machine may operate in the capacity of a server or a client user machine in server-client user network environment, or as a peer machine in a peer-to-peer (or distributed) network environment. The machine may comprise a server computer, a client user computer, a personal computer (PC), a tablet PC, a laptop computer, a desktop computer, a control system, a network router, switch or bridge, or any machine capable of executing a set of instructions (sequential or otherwise) that specify actions to be taken by that machine. Further, while a single machine is illustrated, the term "machine" shall also be taken to include any collection of machines that individually or jointly execute a set (or multiple sets) of instructions to perform any one or more of the methodologies discussed herein.

The computer system 600 may include a processor 602 (e.g., a central processing unit (CPU), a graphics processing unit (GPU, or both), a main memory 604 and a static memory 604, which communicate with each other via a bus 608. The computer system 600 may further include a video display unit 610 (e.g., a liquid crystal display (LCD), a flat panel, a solid state display, or a cathode ray tube (CRT)). The computer system 600 may include an input device 612 (e.g., a keyboard), a cursor control device 614 (e.g., a mouse), a disk drive unit 616, a signal generation device 618 (e.g., a speaker or remote control) and a network interface device 620.

The disk drive unit 616 may include a machine-readable medium 622 on which is stored one or more sets of instructions 624 (e.g., software) embodying any one or more of the methodologies or functions described herein, including those methods illustrated above. The instructions 624 may also reside, completely or at least partially, within the main memory 604, the static memory 606, or within the processor 602, or a combination thereof, during execution thereof by the computer system 600. The main memory 604 and the processor 602 also may constitute machine-readable media.

Dedicated hardware implementations including, but not limited to, application specific integrated circuits, programmable logic arrays and other hardware devices can likewise be constructed to implement the methods described herein. Applications that may include the apparatus and systems of various embodiments broadly include a variety of electronic and computer systems. Some embodiments implement functions in two or more specific interconnected hardware modules or devices with related control and data signals communicated between and through the modules, or as portions of an application-specific integrated circuit. Thus, the example system is applicable to software, firmware, and hardware implementations.

In accordance with various embodiments of the present disclosure, the methods described herein are intended for operation as software programs running on a computer processor. Furthermore, software implementations can include, but not limited to, distributed processing or component/object distributed processing, parallel processing, or virtual machine processing can also be constructed to implement the methods described herein.

The present disclosure contemplates a machine readable medium 622 containing instructions 624 so that a device connected to the communications network 635, other network, or both, can send or receive voice, video or data, and to communicate over the communications network 635, other network, or both, using the instructions. The instructions 624 may further be transmitted or received over the communications network 635, other network, or both, via the network interface device 620.

While the machine-readable medium 422 is shown in an example embodiment to be a single medium, the term "machine-readable medium" should be taken to include a single medium or multiple media (e.g., a centralized or distributed database, and/or associated caches and servers) that store the one or more sets of instructions. The term "machine-readable medium" shall also be taken to include any medium that is capable of storing, encoding or carrying a set of instructions for execution by the machine and that cause the machine to perform any one or more of the methodologies of the present disclosure.

The terms "machine-readable medium" or "machine-readable device" shall accordingly be taken to include, but not be limited to: memory devices, solid-state memories such as a memory card or other package that houses one or more read-only (non-volatile) memories, random access memories, or other re-writable (volatile) memories; magneto-optical or optical medium such as a disk or tape; or other self-contained information archive or set of archives is considered a distribution medium equivalent to a tangible storage medium. The "machine-readable medium" or "machine-readable device" may be non-transitory in nature. Accordingly, the disclosure is considered to include any one or more of a machine-readable medium or a distribution medium, as listed herein and including art-recognized equivalents and successor media, in which the software implementations herein are stored.

In the specification the terms "comprise, comprises, comprised and comprising" or any variation thereof and the terms include, includes, included and including" or any variation thereof are considered to be totally interchangeable and they should all be afforded the widest possible interpretation and vice versa.

**Claims**

1.  A method of determining or predicting identity of a query cell comprising the steps of:

    - simultaneously incubating the query cell with at least three chemical cell stressor molecules individually in a well of a microtitre plate for 1 to 4 days, in which each chemical cell stressor molecule is provided at a concentration of 0.5 to 2.0 $IC_{50}$; wherein the cell stressor is a molecule or a compound that causes a reduction in cell growth via one or more of multiple cellular pathways, for example pathways involved in cell growth or metabolism;
    - determining the growth response of the query cell in the presence of each of the at least three chemical cell stressor molecules to generate a query cell-specific growth response fingerprint;
    - comparing the query cell-specific growth response fingerprint with one or more reference cell-specific growth response fingerprints corresponding to one or more cells having known identity; and
    - determining or predicting identity of the cell based on the level of correlation between the query cell-specific growth response fingerprint and the one or more reference cell-specific growth response fingerprints.

2.  A method as claimed in any preceding Claim in which the cell is a mammalian producer cell.

3.  A method as claimed in Claim 2 for identifying a specific CHO cell type from a panel of closely related CHO cell types.

4.  A method as claimed in any preceding Claim in which the step of comparing the query cell-specific growth response fingerprint with reference cell-specific growth response fingerprints comprises mathematical modelling, visual comparison, or pattern recognition.

5.  A system suitable for identifying a cell according to a method of Claim 1, the system comprising:

    - a microtiter plate comprising a plurality of wells;
    - at least three individual chemical cell stressor molecules disposed individually within the wells of the microtiter plate;
    - a determination system for determining the growth response of the cell in the presence of each of the three individual chemical cell stressor molecules provided at a concentration of 0.5 to 2 x IC50; wherein the cell stressor is a molecule or a compound that causes a reduction in cell growth via one or more of multiple cellular pathways, for example pathways involved in cell growth or metabolism;
    - a storage system for storing a cell-specific growth response fingerprint corresponding to the at least three growth responses of the cell;
    - a comparison system configured to compare the cell specific growth response fingerprint with one or more reference cell-specific growth response fingerprints, in which the comparison system comprises a computational model configured to input a growth response fingerprint from a query cell, compare the growth response fingerprint with one or more reference growth response fingerprints, and output a content based in part of the comparison result; and
    - a display system for displaying an output of the comparison step.

6.  A system as claimed in Claim 5 in which the determination system comprises a microtitre plate reader configured to detect growth of cells in the wells of the microtitre plate.

7.  A method of generating a reference growth response fingerprint corresponding to a specific cell, comprising the steps of:

    - simultaneously incubating the cell with at least three individual chemical cell stressor molecules in wells of a microtitre plate for 1 to 4 days at a concentration of 0.5 to 2 x IC50; and
    - determining the growth response of the cell in the presence of each of the at least three chemical cell stressor molecules to generate a reference growth response fingerprint.

8.  A method of generating a panel of reference growth response fingerprints corresponding to a panel of distinct cells, comprising the steps of:

    - generating a reference growth response fingerprint for each cell in the panel of cells according to a method of Claim 7; and
    - storing the panel of reference growth response fingerprints,

9. A method according to Claim 8, in which the panel of distinct cells is a panel of clonal cells derived from a single parental cell population.

10. A rapid, high-throughput, computer implemented method of predicting relative fed batch performance of a panel of clonal cells derived from a single parental host cell population, the method comprising the steps of:

- simultaneously assaying a level of growth of each clone in the presence and absence of at least three chemical cell stressor molecules individually in wells of a microtitre plate for 1 to 4 days, in which each chemical cell stressor molecule is provided at a concentration of 0.5 to 2.0 $IC_{50}$; wherein the cell stressor is a molecule or a compound that causes a reduction in cell growth via one or more of multiple cellular pathways, for example pathways involved in cell growth or metabolism;
- comparing the level of growth of each clone in the presence and absence of each of the three chemical cell stressor molecules to provide a normalised growth response value for each clone in each of the three stressed microenvironments;
- inputting a clone-specific growth response fingerprint comprising the normalised growth response value for each clone in each of the stressed microenvironments into a computational model, in which the computational model is generated from growth response fingerprints obtained from a calibration set of clones with known fed batch performance, wherein the computational model is configured to output the predicted fed batch performance value for each clone to predict the relative fed batch performance of the panel of clonal cells.

11. A computer implemented method according to Claim 10, in which the multiple linear computational model employs a least squares solution to fit growth response values to predicted fed batch performance.

12. A computer implemented system configured to predict a relative fed batch performance of a panel of clonal cells derived from a single parental host cell population, the system comprising:

- a determination system to assay a level of growth of each clone in the presence and absence of a plurality of individual chemical cell stressors provided at a concentration of 0.5 to 2.0 IC50 to provide a normalised growth response value for each clone in a plurality of stressed microenvironments; wherein the cell stressor is a molecule or a compound that causes a reduction in cell growth via one or more of multiple cellular pathways, for example pathways involved in cell growth or metabolism; and
- a computational model adapted to process a clone-specific growth response fingerprint comprising the normalised growth response value for each clone in each of the stressed microenvironments, in which the computational model is generated from growth response fingerprints obtained from a calibration set of clones with known fed batch performance, wherein the computational model is configured to output the predicted fed batch performance value for each clone to predict the relative fed batch performance of the panel of clonal cells, and
- means for outputting the predicted fed batch performance value for each clone to predict the relative fed batch performance of the panel of clonal cells.

13. A computer implemented system as claimed in Claim 12, in which the determination system comprises a multiplate reader.

14. A computer implemented system as claimed in Claim 12 or 13 in which the computational model is a multiple linear computational model

**Patentansprüche**

1. Verfahren zum Bestimmen oder Vorhersagen der Identität einer abzuklärenden Zelle, das die folgenden Schritte umfasst:

- gleichzeitiges Inkubieren der abzuklärenden Zelle mit mindestens drei chemischen Zellstressormolekülen einzeln in einem Well einer Mikrotiterplatte für 1 bis 4 Tage, in welchem jedes chemische Zellstressormolekül in einer Konzentration von 0,5 bis 2,0 $IC_{50}$ bereitgestellt ist; wobei der Zellstressor ein Molekül oder eine Verbindung ist, das/die eine Reduzierung des Zellwachstums über einen oder mehrere der zahlreichen zellulären Wege bewirkt, beispielsweise Wege, die am Zellwachstum oder Metabolismus beteiligt sind;
- Bestimmen der Wachstumsreaktion der abzuklärenden Zelle in Gegenwart von jedem der mindestens drei chemischen Zellstressormoleküle, um einen Fingerabdruck der für die abzuklärende Zelle spezifischen Wachstumsreaktion zu erzeugen;
- Vergleichen des Fingerabdrucks der für die abzuklärende Zelle spezifischen Wachstumsreaktion mit einem oder mehreren Fingerabdrücken der für eine Referenzzelle spezifischen Wachstumsreaktion, die der einen oder den mehreren Zellen, die eine bekannte Identität aufweisen, entsprechen; und
- Bestimmen oder Vorhersagen der Identität der Zelle basierend auf der Höhe der Korrelation zwischen dem Fingerabdruck der für die abzuklärende Zelle spezifischen Wachstumsreaktion und dem einen oder den mehreren Fingerabdrücken der für eine Referenzzelle spezifischen Wachstumsreaktion.

**2.** Verfahren nach dem vorstehenden Anspruch, in welchem die Zelle eine Säugerproduktionszelle ist.

**3.** Verfahren nach Anspruch 2 für das Identifizieren eines spezifischen CHO-Zelltyps aus einer Gruppe von eng verwandten CHO-Zelltypen.

**4.** Verfahren nach einem vorstehenden Anspruch, in welchem der Schritt des Vergleichens des Fingerabdrucks der für die abzuklärende Zelle spezifischen Wachstumsreaktion mit den Fingerabdrücken der für eine Referenzzelle spezifischen Wachstumsreaktion mathematische Modellierung, visuellen Vergleich oder Mustererkennung umfasst.

**5.** System, das für das Identifizieren einer Zelle nach einem Verfahren nach Anspruch 1 geeignet ist, wobei das System Folgendes umfasst:

- eine Mikrotiterplatte, die eine Vielzahl von Wells umfasst;
- mindestens drei einzelne chemische Zellstressormoleküle, die einzeln innerhalb der Wells der Mikrotiterplatte eingebracht sind;
- ein Bestimmungssystem für das Bestimmen der Wachstumsreaktion der Zelle in Gegenwart von jedem der drei einzelnen chemischen Zellstressormoleküle, die in einer Konzentration von 0,5 bis 2 x IC50 bereitgestellt sind; wobei der Zellstressor ein Molekül oder eine Verbindung ist, das/die eine Reduzierung des Zellwachstums über einen oder mehrere der zahlreichen zellulären Wege bewirkt, beispielsweise Wege, die am Zellwachstum oder Metabolismus beteiligt sind;
- ein Speichersystem für das Speichern eines Fingerabdrucks der für eine Zelle spezifischen Wachstumsreaktion, der den mindestens drei Wachstumsreaktionen der Zelle entspricht;
- ein Vergleichssystem, das zum Vergleichen des Fingerabdrucks der für eine Zelle spezifischen Wachstumsreaktion mit einem oder mehreren Fingerabdrücken der für eine Referenzzelle spezifischen Wachstumsreaktion konfiguriert ist, in welchem das Vergleichssystem ein Rechenmodell umfasst, das für Folgendes konfiguriert ist: Eingeben eines Fingerabdrucks der Wachstumsreaktion von einer abzuklärenden Zelle, Vergleichen des Fingerabdrucks der Wachstumsreaktion mit einem oder mehreren Referenz-Fingerabdrücken der Wachstumsreaktion und Ausgeben eines Inhalts basierend teilweise auf dem Vergleichsergebnis; und
- eine Anzeigesystem für das Anzeigen einer Ausgabe des Vergleichsschritts.

**6.** System nach Anspruch 5, in welchem das Bestimmungssystem ein Mikrotiterplatten-Lesegerät umfasst, das zur Detektion von Wachstum von Zellen in den Wells der Mikrotiterplatte konfiguriert ist.

**7.** Verfahren zum Erzeugen eines Referenz-Fingerabdrucks der Wachstumsreaktion, der einer spezifischen Zelle entspricht, das die folgenden Schritte umfasst:

- gleichzeitiges Inkubieren der Zelle mit mindestens drei einzelnen chemischen Zellstressormolekülen in Wells einer Mikrotiterplatte für 1 bis 4 Tage in einer Konzentration von 0,5 bis 2 x IC50; und
- Bestimmen der Wachstumsreaktion der Zelle in Gegenwart von jedem der mindestens drei chemischen Zellstressormoleküle, um einen Referenz-Fingerabdruck der Wachstumsreaktion zu erzeugen.

**8.** Verfahren zum Erzeugen einer Gruppe von Referenz-Fingerabdrücken der Wachstumsreaktion, die einer Gruppe von verschiedenen Zellen entspricht, das die folgenden Schritte umfasst:

- Erzeugen eines Referenz-Fingerabdrucks der Wachstumsreaktion für jede Zelle in der Gruppe von Zellen nach einem Verfahren nach Anspruch 7; und
- Speichern der Gruppe von Referenz-Fingerabdrücken der Wachstumsreaktion.

**9.** Verfahren nach Anspruch 8, in welchem die Gruppe von verschiedenen Zellen eine Gruppe von klonalen Zellen ist, die von einer einzigen parentalen Zellpopulation abstammen.

**10.** Schnelles, computerimplementiertes Hochdurchsatzverfahren zum Vorhersagen von relativer Fed-Batch-Leistung einer Gruppe von klonalen Zellen, die von einer einzigen parentalen Wirtszellpopulation abstammen, wobei das Verfahren die folgenden Schritte umfasst:

- gleichzeitiges Untersuchen einer Höhe des Wachstums von jedem Klon in Gegenwart und Abwesenheit von mindestens drei chemischen Zellstressormolekülen einzeln in Wells einer Mikrotiterplatte für 1 bis 4 Tage, in welchem jedes chemische Zellstressormolekül in einer Konzentration von 0,5 bis 2,0 $IC_{50}$ bereitgestellt ist; wobei der Zellstressor ein Molekül oder eine Verbindung ist, das/die eine Reduzierung des Zellwachstums über einen oder mehrere der zahlreichen zellulären Wege bewirkt, beispielsweise Wege, die am Zellwachstum oder Metabolismus beteiligt sind;
- Vergleichen der Höhe des Wachstums von jedem Klon in Gegenwart und Abwesenheit von jedem der drei chemischen Zellstressormoleküle, um einen normalisierten Wert der Wachstumsreaktion für jeden Klon in jeder der drei gestressten Mikroumgebungen bereitzustellen;
- Eingeben eines klonspezifischen Fingerabdrucks der Wachstumsreaktion, der den normalisierten Wert der Wachstumsreaktion für jeden Klon in jeder der gestressten Mikroumgebungen umfasst, in ein Rechenmodell, in welchem das Rechenmodell aus Fingerabdrücken der Wachstumsreaktion erzeugt wird, die von einem Kalibriersatz von Klonen mit bekannter Fed-Batch-Leistung erhalten wurden, wobei das Rechenmodell zum Ausgeben des vorhergesagten Fed-Batch-Leistungswerts für jeden Klon konfiguriert ist, um die relative Fed-Batch-Leistung der Gruppe von klonalen Zellen vorherzusagen.

**11.** Computerimplementiertes Verfahren nach Anspruch 10, in welchem das multiple lineare Rechenmodell eine Lösung der kleinsten Quadrate einsetzt, um Werte der Wachstumsreaktion der vorhergesagten Fed-Batch-Leistung anzupassen.

**12.** Computerimplementiertes System, das zur Vorhersage einer relativen Fed-Batch-Leistung einer Gruppe von klonalen Zellen konfiguriert ist, die von einer einzigen parentalen Wirtszellpopulation abstammen, wobei das System Folgendes umfasst:

- ein Bestimmungssystem zum Untersuchen einer Höhe des Wachstums von jedem Klon in Gegenwart und Abwesenheit einer Vielzahl von einzelnen chemischen Zellstressoren, die in einer Konzentration von 0,5 bis 2,0 IC50 bereitgestellt sind, um einen normalisierten Wert der Wachstumsreaktion für jeden Klon in einer Vielzahl von gestressten Mikroumgebungen bereitzustellen; wobei der Zellstressor ein Molekül oder eine Verbindung ist, das/die eine Reduzierung des Zellwachstums über einen oder mehrere der zahlreichen zellulären Wege bewirkt, beispielsweise Wege, die am Zellwachstum oder Metabolismus beteiligt sind; und
- ein Rechenmodell, das zum Verarbeiten eines klonspezifischen Fingerabdrucks der Wachstumsreaktion, der den normalisierten Wert der Wachstumsreaktion für jeden Klon in jeder der gestressten Mikroumgebungen umfasst, geeignet ist, in welchem das Rechenmodell aus Fingerabdrücken der Wachstumsreaktion erzeugt wird, die von einem Kalibriersatz von Klonen mit bekannter Fed-Batch-Leistung erhalten wurden, wobei das Rechenmodell zum Ausgeben des vorhergesagten Fed-Batch-Leistungswerts für jeden Klon konfiguriert ist, um die relative Fed-Batch-Leistung der Gruppe von klonalen Zellen vorherzusagen, und
- Mittel für das Ausgeben des vorhergesagten Fed-Batch-Leistungswerts für jeden Klon, um die relative Fed-Batch-Leistung der Gruppe von klonalen Zellen vorherzusagen.

**13.** Computerimplementiertes System nach Anspruch 12, in welchem das Bestimmungssystem ein Multiplatten-Lesegerät umfasst.

**14.** Computerimplementiertes System nach Anspruch 12 oder 13, in welchem das Rechenmodell ein multiples lineares Rechenmodell ist.

**Revendications**

**1.** Méthode destinée à la détermination ou la prédiction de l'identité d'une cellule requête, comprenant les étapes consistant à :

- incuber de manière simultanée la cellule requête avec au moins trois molécules chimiques facteurs de stress cellulaire, individuellement dans un puits d'une plaque de microtitration pendant de 1 à 4 jours, où chaque molécule chimique facteur de stress cellulaire est fournie selon une concentration allant de 0,5 à 2,0 $CI_{50}$; où

le facteur de stress cellulaire est une molécule ou un composé qui provoque une réduction de la croissance cellulaire via une ou plusieurs parmi de multiples voies cellulaires, par exemple des voies impliquées dans la croissance ou le métabolisme cellulaire ;

- déterminer la réponse de croissance de la cellule requête en présence de chacune parmi les au moins trois molécules chimiques facteurs de stress cellulaire, afin de générer une empreinte de réponse de croissance spécifique de cellule requête ;

- comparer l'empreinte de réponse de croissance spécifique de cellule requête avec une ou plusieurs empreintes de réponse de croissance spécifique de cellule de référence correspondant à une ou plusieurs cellules ayant des identités connues ; et

- déterminer ou prédire l'identité de la cellule, sur la base du taux de corrélation entre l'empreinte de réponse de croissance spécifique de cellule requête et les une ou plusieurs empreintes de réponse de croissance spécifique de cellule de référence.

2. Méthode selon la revendication précédente, dans laquelle la cellule est une cellule productrice mammalienne.

3. Méthode selon la revendication 2, destinée à l'identification d'un type de cellule CHO spécifique à partir d'un panel de types de cellule CHO étroitement apparentés.

4. Méthode selon l'une quelconque des revendications précédentes, dans laquelle l'étape consistant à comparer l'empreinte de réponse de croissance spécifique de cellule requête avec des empreintes de réponse de croissance spécifique de cellule de référence comprend une modélisation mathématique, une comparaison visuelle, ou une reconnaissance des formes.

5. Système convenable pour l'identification d'une cellule selon une méthode selon la revendication 1, le système comprenant :

- une plaque de microtitration comprenant une pluralité de puits ;
- au moins trois molécules chimiques individuelles facteurs de stress cellulaire, disposées individuellement au sein des puits de la plaque de microtitration ;
- un système de détermination destiné à la détermination de la réponse de croissance de la cellule en présence de chacune parmi les trois molécules chimiques individuelles facteurs de stress cellulaire, fournies selon une concentration allant de 0,5 à 2 $CI_{50}$; où le facteur de stress cellulaire est une molécule ou un composé qui provoque une réduction de la croissance cellulaire via une ou plusieurs parmi de multiples voies cellulaires, par exemple des voies impliquées dans la croissance ou le métabolisme cellulaire ;
- un système de stockage destiné au stockage d'une empreinte de réponse de croissance spécifique de cellule correspondant au au moins trois réponses de croissance de la cellule ;
- un système de comparaison configuré afin de comparer l'empreinte de réponse de croissance spécifique de cellule avec une ou plusieurs empreintes de réponse de croissance spécifique de cellule de référence, le système de comparaison comprenant un modèle informatique configuré pour saisir une empreinte de réponse de croissance issue d'une cellule requête, comparer l'empreinte de réponse de croissance avec une ou plusieurs empreintes de réponse de croissance de référence, et sortir une teneur basée en partie sur le résultat de comparaison ; et
- un système d'affichage destiné à afficher une sortie de l'étape de comparaison.

6. Système selon la revendication 5, dans lequel le système de détermination comprend un lecteur de plaques de microtitration configuré afin de détecter la croissance de cellules dans les puits de la plaque de microtitration.

7. Méthode destinée à la génération d'une empreinte de réponse de croissance de référence correspondant à une cellule spécifique, comprenant les étapes consistant à :

- incuber de manière simultanée la cellule avec au moins trois molécules chimiques individuelles facteurs de stress cellulaire, dans des puits d'une plaque de microtitration pendant de 1 à 4 jours, selon une concentration allant de 0,5 à 2 $CI_{50}$; et
- déterminer la réponse de croissance de la cellule en présence de chacune parmi les au moins trois molécules chimiques facteurs de stress cellulaire, afin de générer une empreinte de réponse de croissance de référence.

8. Méthode de génération d'un panel d'empreintes de réponse de croissance de référence correspondant à un panel de cellules distinctes, comprenant les étapes consistant à :

- générer une empreinte de réponse de croissance de référence pour chaque cellule dans le panel de cellules selon la méthode selon la revendication 7 ; et
- stocker le panel d'empreintes de réponse de croissance de référence.

9. Méthode selon la revendication 8, dans laquelle le panel de cellules distinctes est un panel de cellules clonales dérivées d'une population de cellules parentales uniques.

10. Méthode mise en œuvre par ordinateur, rapide et à haut débit, destinée à la prédiction des performances de culture discontinue alimentée relatives d'un panel de cellules clonales dérivées d'une population de cellules hôtes parentales uniques, la méthode comprenant les étapes consistant à :

- doser de manière simultanée un taux de croissance de chaque clone en présence et en l'absence d'au moins trois molécules chimiques facteurs de stress cellulaire, individuellement dans des puits d'une plaque de microtitration pendant de 1 à 4 jours, où chaque molécule chimique facteur de stress cellulaire est fournie selon une concentration allant de 0,5 à 2,0 $CI_{50}$ ; où le facteur de stress cellulaire est une molécule ou un composé qui provoque une réduction de la croissance cellulaire via une ou plusieurs parmi de multiples voies cellulaires, par exemple des voies impliquées dans la croissance ou le métabolisme cellulaire ;
- comparer le taux de croissance de chaque clone en présence et en l'absence de chacune des trois molécules chimiques facteurs de stress cellulaire, afin de fournir une valeur de réponse de croissance normalisée pour chaque clone dans chacun des trois micro-environnements stressés ;
- saisir une empreinte de réponse de croissance spécifique de clone, comprenant la valeur de réponse de croissance normalisée pour chaque clone dans chacun des micro-environnements stressés dans un modèle informatique, où le modèle informatique est généré à partir des empreintes de réponse de croissance obtenues à partir d'un set d'étalonnage de clones ayant des performances de culture discontinue alimentée connues, où le modèle informatique est configuré pour sortir la valeur de performances de culture discontinue alimentée prédite pour chaque clone afin de prédire les performances de culture discontinue alimentée relatives du panel de cellules clonales.

11. Méthode mise en œuvre par ordinateur selon la revendication 10, dans laquelle le modèle informatique linéaire multiple emploie une méthode des moindres carrés pour ajuster les valeurs de réponse de croissance aux performances de culture discontinue alimentée prédites.

12. Système mis en œuvre par ordinateur, configuré afin de prédire des performances de culture discontinue alimentée relatives d'un panel de cellules clonales dérivées d'une population de cellules hôtes parentales uniques, le système comprenant :

- un système de détermination destiné à doser le taux de croissance de chaque clone en présence et en l'absence d'une pluralité de facteurs de stress cellulaire chimiques individuels, fournis selon une concentration allant de 0,5 à 2,0 $CI_{50}$, afin de fournir une valeur de réponse de croissance normalisée pour chaque clone dans une pluralité de micro-environnements stressés ; où le facteur de stress cellulaire est une molécule ou un composé qui provoque une réduction de la croissance cellulaire via une ou plusieurs parmi de multiples voies cellulaires, par exemple des voies impliquées dans la croissance ou le métabolisme cellulaire ; et
- un modèle informatique adapté afin de traiter une empreinte de réponse de croissance spécifique de clone, comprenant la valeur de réponse de croissance normalisée pour chaque clone dans chacun des micro-environnements stressés, où le modèle informatique est généré à partir des empreintes de réponse de croissance obtenues à partir d'un set d'étalonnage de clones ayant des performances de culture discontinue alimentée connues, où le modèle informatique est configuré pour sortir la valeur de performances de culture discontinue alimentée prédite pour chaque clone afin de prédire les performances de culture discontinue alimentée relatives du panel de cellules clonales ; et
- un moyen destiné à sortir la valeur de performances de culture discontinue alimentée prédite pour chaque clone afin de prédire les performances de culture discontinue alimentée relatives du panel de cellules clonales.

13. Système mis en œuvre par ordinateur selon la revendication 12 , dans lequel le système de détermination comprend un lecteur multi-plaques.

14. Système mis en œuvre par ordinateur selon la revendication 12 ou 13 , dans lequel le modèle informatique est un modèle informatique linéaire multiple.

**FIG. 1**

**FIG. 2**

**Fig. 3**

Cluster Dendrogram

All perfect clustering of groups of cell types

**FIG. 4**

Group 1

Group 2

This vector maximises the ratio of the 'between groups variance' to the 'within groups variance'

Chemical response 2

Chemical response 1

**FIG. 5**

Distance along vector can be approximated by Gaussian distributions and thus the group probability of a new point can be calculated from the distance along the separation vector

Group 1          Group 2

## FIG. 6

Negligible variance
In this dimension

Strong correlation

Line=
a(Chem1)+b(chem2)

Chemical 1 response

Chemical 2 response

Line=
a(Chem1)+b(chem2)

Data can be expressed in a 1 dimensional
format without substantial loss of information

Line=
a(Chem1)+b(chem2)

Data reduced from a 2 dimensional space to a 1 dimensional space

## FIG. 7

fit

## FIG. 8

Actual group

100%
Correct predictions

**FIG. 9**

**FIG. 10**

**FIG. 11**

**bootstrap cross validation**

**FIG. 12**

600

608

| Processor | |
|---|---|
| Instructions | |
| 602 | |

624

| Main Memory | |
|---|---|
| Instructions | |
| 604 | |

624

| Static Memory | |
|---|---|
| Instructions | |
| 606 | |

624

| Network |
|---|
| Interface |
| 620 |
| Device |

635
Communications
Network

| Video |
|---|
| Display 610 |

| Alpha-Numeric |
|---|
| Input       612 |
| Device |

| Cursor Control |
|---|
| Device 614 |

| Machine- |
|---|
| Readable |
| Medium |
| Instructions |
| 622 |
| 616 |

624

| Signal Generation |
|---|
| Device     618 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RACHEL LEGMANN et al.** A Strategy for clone selection under different production conditions. *Biotechnology Progress,* 29 May 2011, vol. 27 (3), 757-765

- Evaluation of Action Mechansims of Toxic Chemicals Using JFCR39, a Panel of Human Cancer Cell Lines. **N. NAKATSU et al.** Molecular Pharmacology. The American Society for Pharmacology and Experimental Therapeutics, 03 August 2007, vol. 72, 1171-1180
- *Nature Biotechnology,* 2004, vol. 22, 1393-1398